# EUROPEAN PATENT APPLICATION

(11) **EP 3 401 328 A1**
(43) Date of publication of application: **14.11.2018**
(21) Application number: 17170445.5
(22) Date of filing: 10.05.2017
(51) Int. Cl.: C07K 16/00, C07K 16/46, C12N 15/63

(54) **ONE STEP ANTIBODY HUMANIZATION BY GOLDEN GATE BASED GERMLINE FRAMEWORK REGION SHUFFLING**

(71) Applicant: Bayer Pharma Aktiengesellschaft, 13353 Berlin (DE)
(72) Inventor: WEBER, Ernst, 40764 Langenfeld (DE); BIRKENFELD, Jörg, 60486 Frankfurt am Main (DE)
(74) Representative: BIP Patents

(57) **Abstract**

This invention provides a method of producing a humanized antibody that immunospecifically binds to an antigen comprising the method of a novel rapid identification of optimal human germline framework region (FR) combinations, capable of maintaining structural and functional features of non-human CDRs. without structural guidance. This method solves the problem of an increased need for an antibody humanization platform technology compatible with robust and reliable high throughput antibody engineering workflows, by providing sets of defined and reusable FR entities which can be assembled without the use of oligonucleotide and PCR based technologies which introduce unwanted amino acid exchanges.

## Description

### FIELD OF THE INVENTION

This invention is related to a novel method for rapid identification of optimal human germline framework region (FR) combinations, capable of maintaining structural and functional features of non-human CDRs. without structural guidance. This method solves the problem of an increased need for an antibody humanization platform technology compatible with robust and reliable high throughput antibody engineering workflows, by providing sets of defined and reusable FR entities which can be orderly assembled with non-human CDRs to produce humanized antibodies without the use of oligonucleotide and PCR based technologies which introduce unwanted amino acid exchanges.

### BACKGROUND OF THE INVENTION

Antibodies may be generated using hybridoma technologies (for example see Köhler and Milstein Nature. 1975 Aug 7;256(5517):495-7) or B-cell cloning technologies, resulting in for example murine, rat, or rabbit antibodies which can be converted into chimeric or humanized antibodies. Humanization has played a fundamental role in the remarkable progress of antibodies as therapeutic reagents because immunogenicity is a critical concern when developing an antibody-based drug.

Only defined parts of the variable region (VR) of antibodies participate directly in the binding of antigen. Studies have shown that variability in the VRs of both L and H chains is for the most part restricted to three small hypervariable regions (also called complementarity determining regions, or CDRs) in each chain. The remaining parts of the variable region, known as framework regions (FR), are relatively constant.

Antibody humanization methods are designed to minimize deviation from human germline sequences to reduce the risk of immunogenicity when applied to humans, while retaining the specificity and affinity of the parental non-human antibody. In general two main trends in the field can be distinguished: rational and empirical methods to humanize antibodies.

Many groups have devised protein engineering techniques to decrease the risk for immunogenicity of therapeutic antibodies. Traditionally, the non-human donor CDRs are combined with human framework regions (CDR-grafting). To identify well matched combinations of human FRs and non-human CDRs, usually a human antibody template sequence, preferentially selected from the human germline repertoire is selected by the degree of homology to the donor antibody sequence, i.e. that the most homologous human antibody to the non-human antibody in the variable region is used as the template for humanization. The rationale is that the FR sequences serve to maintain the CDRs in their correct spatial orientation for interaction with an antigen, and that framework residues can sometimes even participate in antigen binding. Thus, if the selected human FR sequences are most similar to the sequences of the donor FRs, it will maximize the likelihood that affinity and specificity will be retained in the humanized antibody.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Natl Acad. Sci. USA 86:10029-10033 (1989); US Patent Nos. 5,821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall' Acqua et al., Methods 36:43-60 (2005) (describing "FR shuffling"); and Osboum et al., Methods 36:61-68 (2005) and Klimka et al., Br. J. Cancer, 83:252-260 (2000) (describing the "guided selection" approach to FR shuffling).

Although a humanized antibody is most likely less immunogenic than its natural or chimeric counterpart in a human, many groups find that CDR-grafted humanized antibody display significantly decreased binding affinity (e.g., Riechmann et al., Nature 332:323-329 (1988)). Further mutations were necessary to recover the binding affinity of the original non-human antibody. Therefore in addition to classical CDR-grafting, phage display and high-throughput screening (HTS) techniques have emerged as efficient tools to explore combinatorial libraries of millions and billions of antibody variants and select functional ones. Furthermore structural modeling was used to identify framework amino acid residues which were likely to interact with the CDRs or antigen, and mouse amino acids were used at these positions in the humanized antibody. But the task of antibody humanization is by far a standard procedure.

US 2003/0040606 discloses a framework patching approach, in which the variable region of the immunoglobulin is compartmentalized into FR1, CDR1, FR2, CDR2, FR3, CDR3 and FR4, and the individual FR sequence is selected by the best homology between the non-human antibody and the human antibody template. This approach, however, is labor intensive, and the optimal framework regions may not be easily identified.

WO 2005/042743 A2, WO 2006/102095 A2 and Dall'Acqua, et al. Methods. 2005 May, 36(1):43-60 disclose a framework region (FR) shuffling approach for humanization. In FR shuffling whole FRs are combined with the non-human CDRs instead of creating combinatorial libraries of selected residue variants. All six non-human CDRs are cloned into a library of human germline FRs. This multistep antibody humanization technique uses separate vectors for heavy and light chain in combination with PCR technologies. In this approach the coherence of recombinatorial heavy and light chain variants is not retained, which complicates the identification of optimal heavy and light chain pairs.

Furthermore as described in WO 2006/102095 A2 and Dall'Acqua, et al.. Methods. 2005 May, 36(1):43-60 the shuffling of FRs and the generation of complete antibody sequences is based on sets of oligonucleotides which are fused by error-prone PCR based technologies, leading to multiple unwanted amino acid exchanges, which have to be corrected and eliminated in subsequent cumbersome cloning steps. A further limitation of the described method is that sets of oligos are donor antibody-specific and thus cannot be re-used in other humanization projects.

Type IIS restriction enzymes in combination with a DNA-ligase allow the PCR-independent, errorless assembly of multiple DNA-fragments. One technology based on this combination of enzymatic activities (restriction enzyme type IIs and DNA ligase acting simultaneously or subsequently) is termed "Golden Gate cloning" (Engler et al. (2009) PLoS One 4: e5553). Type IIS restriction enzymes have the special feature to cleave outside of their recognition site. When these recognition sites are placed to the far 5' and 3' end of any DNA fragment in inverse orientation, they are removed in the cleavage process, allowing two DNA fragments flanked by compatible sequence overhangs to be ligated seamlessly. A detailed definition of restriction enzymes and the features of these enzymes is provides by Roberts et al. (Roberts RJ, et al. (2003) "A nomenclature for restriction enzymes, DNA methyltransferases, homing endonucleases and their genes." Nucleic Acids Res 31: 1805-1812).

This invention provides an improved humanization method based on framework region (FR) shuffling as a solution to the described problems. Using "Golden Gate cloning", defined and sequence quality-controlled; standardized oligonucleotide module sets for each FR can be assembled by non-PCR methods.

### SUMMARY OF THE INVENTION

The present invention provides a method of producing a humanized antibody that immunospecifically binds to an antigen, said method comprising: (a) synthesizing a plurality of polynucleotide sequences each comprising a nucleotide sequence encoding a humanized heavy chain variable region, said nucleotide sequence produced by fusing together nucleic acid sequence encoding a human heavy chain framework region 1, a nucleic acid sequence encoding a heavy chain CDR1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a nonhuman donor antibody heavy chain variable region that immunospecifically binds said antigen and each heavy chain framework region nucleic acid sequence is from a subbank comprising a plurality of nucleic acid sequences encoding said human heavy chain framework region from human germline framework sequences and/or framework regions from functional human antibody sequences; (b) introducing the polynucleotide sequences into a population of cells each containing a polynucleotide sequence encoding a light chain variable region; and (c) expressing the polynucleotide sequences encoding the heavy chain variable region and the light chain variable region, characterized in that the nucleic acid sequences in step (a) are fused together by a step comprising the sequential or simultaneous activities of a type IIS restriction enzyme and a DNA ligase.

In another embodiment the present invention provides a method of producing a humanized antibody that immunospecifically binds to an antigen, said method comprising: (a) synthesizing a plurality of polynucleotide sequences each comprising a nucleotide sequence encoding a humanized light chain variable region, said nucleotide sequence produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a nonhuman donor antibody light chain variable region that immunospecifically binds said antigen and each light chain framework region nucleic acid sequence is from a subbank comprising a plurality of nucleic acid sequences encoding said human light chain framework region from human germline framework sequences and/or framework regions from functional human antibody sequences; (b) introducing the polynucleotide sequences into a population of cells each containing a polynucleotide sequence encoding a heavy chain variable region and (c) expressing the polynucleotide sequences encoding the heavy chain variable region and the light chain variable region, characterized in that the nucleic acid sequences in step (a) are fused together by a step comprising the sequential or simultaneous activities of a type IIS restriction enzyme and a DNA ligase.

In another embodiment the present invention provides a method of producing a humanized antibody that immunospecifically binds to an antigen, said method comprising: (a) synthesizing a plurality of first polynucleotide sequences each comprising a nucleotide sequence encoding a humanized heavy chain variable region, said nucleotide sequence produced by fusing together a nucleic acid sequence encoding a human heavy chain framework region 1, a nucleic acid sequence encoding a heavy chain CDR1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a non-human donor antibody heavy chain variable region that immunospecifically binds said antigen and each heavy chain framework region nucleic acid sequence is from a sub-bank comprising a plurality of nucleic acid sequences encoding said heavy chain framework region from human germline framework sequences and/or framework regions from functional human antibody sequences; (b) synthesizing a plurality of second polynucleotide sequences each comprising a nucleotide sequence encoding a humanized light chain variable region, said nucleotide sequence produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a non-human donor antibody light chain variable region that immunospecifically binds said antigen and each light chain framework region nucleic acid sequence is from a sub-bank comprising a plurality of nucleic acid sequences encoding said human light chain framework region from human germline framework sequences and/or framework regions from functional human antibody sequences; (c) introducing the nucleic acid sequences generated in steps (a) and (b) into a population of cells; and (d) expressing the nucleotide sequences encoding the heavy chain variable region and the light chain variable region, characterized in that the nucleic acid sequences in step (a) and/or (b) are fused together by a step comprising the sequential or simultaneous activities of a type IIS restriction enzyme and a DNA ligase.

In another embodiment the present invention provides a method of producing a humanized antibody that immunospecifically binds to an antigen which further comprises, after the step of expressing the nucleotide sequences, the step of screening for an antibody that immunospecifically binds to the antigen.

### DEFINITIONS

Unless defined otherwise, all technical and scientific terms used herein have the meaning commonly understood by one of ordinary skill in the art to which this invention belongs. The following references, however, can provide one of skill in the art to which this invention pertains with a general definition of many of the terms used in this invention, and can be referenced and used so long as such definitions are consistent with the meaning commonly understood in the art. Such references include, but are not limited to, Singleton et al., Dictionary of Microbiology and Molecular Biology (2nd ed. 1994); The Cambridge Dictionary of Science and Technology (Walker ed., 1988); Hale & Marham, The Harper Collins Dictionary of Biology (1991); and Lackie et al., The Dictionary of Cell & Molecular Biology (3d ed. 1999); and Cellular and Molecular Immunology, Eds. Abbas, Lichtman and Pober, 2nd Edition, W.B. Saunders Company. Any additional technical resource available to the person of ordinary skill in the art providing definitions of terms used herein having the meaning commonly understood in the art can be consulted. For the purposes of the present invention, the following terms are further defined. Additional terms are defined elsewhere in the description. As used herein and in the appended claims, the singular forms "a," and "the" include plural reference unless the context clearly dictates otherwise. Thus, for example, reference to "a gene" is a reference to one or more genes and includes equivalents thereof known to those skilled in the art, and so forth.

The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof.

Amino acids may be referred to herein by their commonly known three letter symbols or by the one-letter symbols recommended by the IUPAC-IUB Biochemical Nomenclature Commission. Nucleotides, likewise, may be referred to by their commonly accepted single-letter codes.

The term "antibody", as used herein, is intended to refer to immunoglobulin molecules, preferably comprised of four polypeptide chains, two heavy (H) chains and two light (L) chains which are typically inter-connected by disulfide bonds. Each heavy chain is comprised of a heavy chain variable region (abbreviated herein as VH) and a heavy chain constant region. The heavy chain constant region can comprise e.g. three domains CH1, CH2 and CH3. Each light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs arranged from amino-terminus to carboxy-terminus e.g. in the following order: FR1, CDR1, FR2, CDR2, FR3, CDR3, FR4 (for VH: FR-H1, CDR-H1, FR-H2, CDR-H2, FR-H3, CDR-H3, FR-H4 and for VL: FR-L1, CDR-L1, FR-L2, CDR-L2, FR-L3, CDR-L3, FR-L4).

As used herein, the term "Complementarity Determining Regions" (CDRs; e.g., CDR1, CDR2, and CDR3) refers to the amino acid residues of an antibody variable domain the presence of which are necessary for antigen binding. Each variable domain typically has three CDR regions identified as CDR1, CDR2 and CDR3 (for VH: CDR-H1, CDR-H2, and CDR-H3 and for VL: CDR-L1, CDR-L2, and CDR-L3. Each complementarity determining region may comprise amino acid residues from a "complementarity determining region" as defined by Kabat (e.g. about residues 24-34 (L1), 50-56 (L2) and 89-97 (L3) in the light chain variable domain and 31-35 (H1), 50-65 (H2) and 95-102 (H3) in the heavy chain variable domain; (Kabat et al., Sequences of Proteins of Immulological Interest, 5th Ed. Public Health Service, National Institutes of Health, Bethesda, MD. (1991)) and/or those residues from a "hypervariable loop" (e.g. about residues 26-32 (L1), 50-52 (L2) and 91-96 (L3) in the light chain variable domain and 26- 32 (H1), 53-55 (H2) and 96-101 (H3) in the heavy chain variable domain (Chothia and Lesk; J Mol Biol 196: 901-917 (1987)). In some instances, a complementarity determining region can include amino acids from both a CDR region defined according to Kabat and a hypervariable loop.

As used herein, the term "framework", "framework region" or "framework sequence" (FR) refers to the remaining sequences of a variable region minus the CDRs. Because the exact definition of a CDR sequence can be determined by different systems, the meaning of a framework sequence is subject to correspondingly different interpretations. The six CDRs (CDR-L1, CDR-L2, and CDR-L3 of light chain and CDR-H1, CDR-H2, and CDR-H3 of heavy chain) also divide the framework regions (FR) on the light chain and the heavy chain into four sub-regions (FR1, FR2, FR3 and FR4) on each chain, in which CDR1 is positioned between FR1 and FR2, CDR2 between FR2 and FR3, and CDR3 between FR3 and FR4 (resulting in the following arrangement for VH: FR-H1, CDR-H1, FR-H2, CDR-H2, FR-H3, CDR-H3, FR-H4 and for VL: FR-L1, CDR-L1, FR-L2, CDR-L2, FR-L3, CDR-L3, FR-L4). Without specifying the particular sub-regions as FR1, FR2, FR3 or FR4, a framework region, as referred by others, represents the combined FR's within the variable region of a single, naturally occurring immunoglobulin chain. As used herein, a FR represents one of the four sub-regions, and FRs represents two or more of the four sub-regions constituting a framework region.

Depending on the amino acid sequence of the constant domain of their heavy chains, intact antibodies can be assigned to different "classes". There are five major classes of intact antibodies: IgA, IgD, IgE, IgG, and IgM, and several of these maybe further divided into "subclasses" (isotypes), e.g., IgG1, IgG2, IgG3, IgG4, IgA1, and IgA2. A preferred class of immunoglobulins for use in the present invention is IgG.

The heavy-chain constant domains that correspond to the different classes of antibodies are called [alpha], [delta], [epsilon], [gamma], and [mu], respectively. The subunit structures and three-dimensional configurations of different classes of immunoglobulins are well known. As used herein antibodies are conventionally known antibodies and functional fragments thereof.

A "functional fragment" or "antigen-binding antibody fragment" of an antibody/immunoglobulin hereby is defined as a fragment of an antibody/immunoglobulin (e.g., a variable region of an IgG) that retains the antigen-binding region. An "antigen-binding region" of an antibody typically is found in one or more hyper variable region(s) of an antibody, e.g., the CDR1, -2, and/or -3 regions; however, the variable "framework" regions can also play an important role in antigen binding, such as by providing a scaffold for the CDRs. Preferably, the "antigen-binding region" comprises at least amino acid residues 4 to 103 of the variable light (VL) chain and 5 to 109 of the variable heavy (VH) chain, more preferably amino acid residues 3 to 107 of VL and 4 to 111 of VH, and particularly preferred are the complete VL and VH chains (amino acid positions 1 to 109 of VL and 1 to 113 of VH; numbering according to WO 97/08320).

"Functional fragments", "antigen-binding antibody fragments", or "antibody fragments" of the invention include but are not limited to Fab, Fab', Fab'-SH, F(ab')₂, and Fv fragments; diabodies; single domain antibodies (DAbs), linear antibodies; single-chain antibody molecules (scFv); and multispecifics, such as bi- and tri-specific, antibodies formed from antibody fragments (C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). An antibody other than a "multi-specific" or "multi-functional" antibody is understood to have each of its binding sites identical. The F(ab')₂ or Fab may be engineered to minimize or completely remove the intermolecular disulfide interactions that occur between the CH1 and CL domains.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

Variants of the antibodies or antigen-binding antibody fragments contemplated in the invention are molecules in which the binding activity of the antibody or antigen-binding antibody fragment is maintained.

"Binding proteins" contemplated in the invention are for example antibody mimetics, such as Affibodies, Adnectins, Anticalins, DARPins, Avimers, Nanobodies (reviewed by Gebauer M. et al., Curr. Opinion in Chem. Biol. 2009; 13:245-255; Nuttall S.D. et al., Curr. Opinion in Pharmacology 2008; 8:608-617).

A "human" antibody or antigen-binding fragment thereof is hereby defined as one that is not chimeric (e.g., not "humanized") and not from (either in whole or in part) a non-human species. A human antibody or antigen-binding fragment thereof can be derived from a human or can be a synthetic human antibody. A "synthetic human antibody" is defined herein as an antibody having a sequence derived, in whole or in part, in silico from synthetic sequences that are based on the analysis of known human antibody sequences. In silico design of a human antibody sequence or fragment thereof can be achieved, for example, by analyzing a database of human antibody or antibody fragment sequences and devising a polypeptide sequence utilizing the data obtained there from. Another example of a human antibody or antigen-binding fragment thereof is one that is encoded by a nucleic acid isolated from a library of antibody sequences of human origin (e.g., such library being based on antibodies taken from a human natural source). Examples of human antibodies include antibodies as described in Söderlind et al., Nature Biotech. 2000, 18:853-856.

As used herein, the term "humanized antibody" is an antibody or a variant, derivative, analog or fragment thereof which immunospecifically binds to an antigen of interest and which comprises a framework (FR) region having substantially the amino acid sequence of a human antibody and a complementarity determining region (CDR) having substantially the amino acid sequence of a non-human antibody. As used herein, the term "substantially" in the context of a CDR refers to a CDR having an amino acid sequence at least 80%, preferably at least 85%, at least 90%, at least 95%, at least 98% or at least 99% identical to the amino acid sequence of a non-human antibody CDR. A humanized antibody comprises substantially all of at least one, and typically two, variable domains (Fab, Fab', F(ab')2, FabC, Fv) in which all or substantially all of the CDR regions correspond to those of a non-human immunoglobulin (i.e., donor antibody) and all or substantially all of the framework regions are those of a human immunoglobulin sequence. Preferably, a humanized antibody also comprises at least a portion of an immunoglobulin constant region (Fc), typically that of a human immunoglobulin. In some embodiments, a humanized antibody contains both the light chain as well as at least the variable domain of a heavy chain. The antibody also may include the CH1, hinge, CH2, CH3, and CH4 regions of the heavy chain. In some embodiments, a humanized antibody only contains a humanized light chain. In some embodiments, a humanized antibody only contains a humanized heavy chain. In specific embodiments, a humanized antibody only contains a humanized variable domain of a light chain and/or humanized heavy chain.

A "chimeric antibody" or antigen-binding fragment thereof is defined herein as one, wherein the variable domains are derived from a non-human origin and some or all constant domains are derived from a human origin.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the term "monoclonal" indicates the character of the antibody as not being a mixture of discrete antibodies. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins. The term "monoclonal" is not to be construed as to require production of the antibody by any particular method. The term monoclonal antibody specifically includes chimeric, humanized and human antibodies.

An "isolated" antibody is one that has been identified and separated from a component of the cell that expressed it. Contaminant components of the cell are materials that would interfere with diagnostic or therapeutic uses of the antibody, and may include enzymes, hormones, and other proteinaceous or nonproteinaceous solutes.

An "isolated" nucleic acid is one that has been identified and separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

As used herein, an antibody "binds specifically to", is "specific to/for" or "specifically recognizes" an antigen of interest, e.g. a tumor-associated polypeptide antigen target, is one that binds the antigen with sufficient affinity such that the antibody is useful as a therapeutic agent in targeting a cell or tissue expressing the antigen, and does not significantly cross-react with other proteins or does not significantly cross-react with proteins other than orthologs and variants (e.g. mutant forms, splice variants, or proteolytically truncated forms) of the aforementioned antigen target. The term "specifically recognizes" or "binds specifically to" or is "specific to/for" a particular polypeptide or an epitope on a particular polypeptide target as used herein can be exhibited, for example, by an antibody, or antigen-binding fragment thereof, having a monovalent K_{D} for the antigen of less than about 10⁻⁴ M, alternatively less than about 10⁻⁵ M, alternatively less than about 10⁻⁶ M, alternatively less than about 10⁻⁷ M, alternatively less than about 10⁻⁸ M, alternatively less than about 10⁻⁹ M, alternatively less than about 10⁻¹⁰ M, alternatively less than about 10⁻¹¹ M, alternatively less than about 10⁻¹² M, or less. An antibody "binds specifically to," is "specific to/for" or "specifically recognizes" an antigen if such antibody is able to discriminate between such antigen and one or more reference antigen(s). In its most general form, "specific binding", "binds specifically to", is "specific to/for" or "specifically recognizes" is referring to the ability of the antibody to discriminate between the antigen of interest and an unrelated antigen, as determined, for example, in accordance with one of the following methods. Such methods comprise, but are not limited to surface plasmon resonance (SPR), Western blots, ELISA-, RIA-, ECL-, IRMA-tests and peptide scans. For example, a standard ELISA assay can be carried out. The scoring may be carried out by standard color development (e.g. secondary antibody with horseradish peroxidase and tetramethyl benzidine with hydrogen peroxide). The reaction in certain wells is scored by the optical density, for example, at 450 nm. Typical background (=negative reaction) may be 0.1 OD; typical positive reaction may be 1 OD. This means the difference positive/negative is more than 5-fold, 10-fold, 50-fold, and preferably more than 100-fold. Typically, determination of binding specificity is performed by using not a single reference antigen, but a set of about three to five unrelated antigens, such as milk powder, BSA, transferrin or the like.

As used herein, the term "immunospecifically binds to an antigen" and analogous terms refer to peptides, polypeptides, proteins (including, but not limited to fusion proteins and antibodies) or fragments thereof that specifically bind to an antigen or a fragment and do not specifically bind to other antigens. A peptide, polypeptide, or protein that immunospecifically binds to an antigen may bind to other antigens with lower affinity as determined by, e.g., immunoassays, BIAcore, or other assays known in the art. Antibodies or fragments that immunospecifically bind to an antigen may be cross-reactive with related antigens. Preferably, antibodies or fragments that immunospecifically bind to an antigen do not cross-react with other antigens.

"Binding affinity" or "affinity" refers to the strength of the total sum of non-covalent interactions between a single binding site of a molecule and its binding partner. Unless indicated otherwise, as used herein, "binding affinity" refers to intrinsic binding affinity which reflects a 1:1 interaction between members of a binding pair (e.g. an antibody and an antigen). The dissociation constant "K_{D}" is commonly used to describe the affinity between a molecule (such as an antibody) and its binding partner (such as an antigen) i.e. how tightly a ligand binds to a particular protein. Ligand-protein affinities are influenced by non-covalent intermolecular interactions between the two molecules. Affinity can be measured by common methods known in the art, including those described herein. In one embodiment, the "K_{D}" or "K_{D} value" according to this invention is measured by using surface plasmon resonance assays using suitable devices including but not limited to Biacore instruments like Biacore T100, Biacore T200, Biacore 2000, Biacore 4000, a Biacore 3000 (GE Healthcare Biacore, Inc.), or a ProteOn XPR36 instrument (Bio-Rad Laboratories, Inc.).

"Percent (%) sequence identity" with respect to a reference polynucleotide or polypeptide sequence, respectively, is defined as the percentage of nucleic acid or amino acid residues, respectively, in a candidate sequence that are identical with the nucleic acid or amino acid residues, respectively, in the reference polynucleotide or polypeptide sequence, respectively, after aligning the sequences and introducing gaps, if necessary, to achieve the maximum percent sequence identity. Conservative substitutions are not considered as part of the sequence identity. Preferred are un-gapped alignments. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

"Sequence homology" indicates the percentage of amino acids that either is identical or that represent conservative amino acid substitutions.

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "Validated FR modules" refers to nucleic acid molecules where the coding DNA sequence of the respective FR, as well as the 5' and 3' fusion sites are validated by sequencing.

The term "Type II restriction endonuclease", "Type II restriction enzyme", "Type II REase", or "Type II enzyme" refers to enzymes that recognize specific DNA sequences and cleave at constant positions at or close to that sequence to produce 5'-phosphates and 3'-hydroxyls.

The term "Type IIA restriction endonuclease", "Type IIA restriction enzyme", "Type IIA REase", or "Type IIA enzyme" refers to a generic designation for any Type II enzymes that recognize asymmetric sequences irrespective of whether they cleave away from the sequence or within the sequence.

The term "Type IIS restriction endonuclease", "Type IIS restriction enzyme", "Type IIS REase", or "Type IIS enzyme" refers to Type IIA enzymes that cleave at least one strand of the DNA duplex outside of the recognition sequence (i.e. cleavage is shifted relative to the recognition sequence). Note that for some enzymes, such as Bsml (recognition sequence: GAATGC), cleavage of the strand written takes place outside of the recognition sequence, whereas cleavage of the complementary strand takes place within the recognition sequence. This is still considered a Type IIS enzyme. Type IIS REases include but are not limited to the well-known enzymes Bsal, Bpil, Esp31, Sap, Lgul.

Bsal is a Type IIS enzyme comprising an amino acid sequence represented by UniProtKB database entry Q6SPF4 (July 5, 2004 - v1). Sap is a Type IIS enzyme comprising an amino acid sequence represented by UniProtKB database entry 052712 (March 29, 2005 - v2). Esp31 is a Type IIS enzyme comprising an amino acid sequence represented by GenBank database entry AAM09641.1.The term "Type IIS cloning method" or "Golden Gate cloning method" refers to methods for fusing together nucleic acid sequences wherein said method comprises the sequential or simultaneous activities of type IIS restriction enzyme/s and a DNA ligase. Among others Type IIS restriction enzymes like Bsal, Bpil, Esp31, Sap, Lgul may be used with the system. A T4 DNA ligase is preferred in this method.

The term "DNA ligase" refers to is a specific type of enzyme (EC 6.5.1.1) that facilitates the joining of DNA strands together by catalyzing the formation of a phosphodiester bond. The T4 DNA ligase is the DNA ligase DNA ligase from bacteriophage T4 (UniProt: P00970; Refseq (Protein): NP_049813.1).

### BRIEF DESCRIPTION OF THE FIGURES

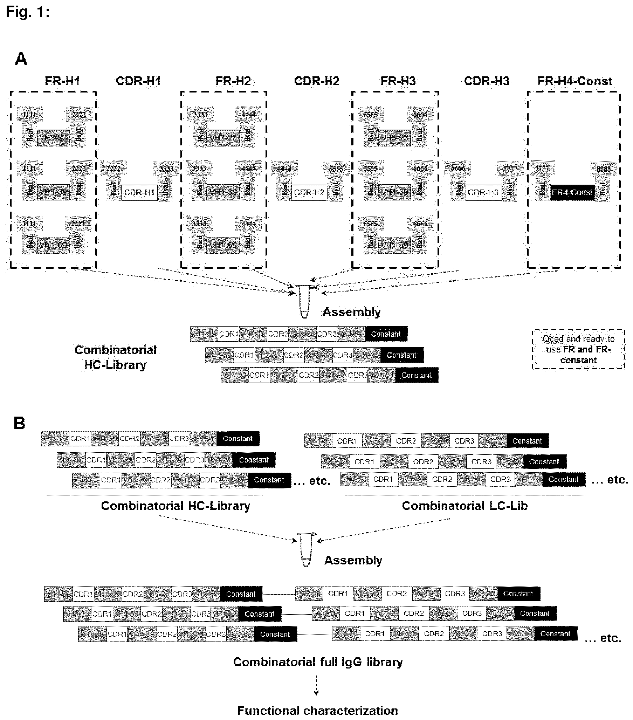

**Fig. 1****:** Overview of complete humanization platform and workflow using sequence validated FR modules.
**A)** As an example only three validated FR modules for each FR position are depicted representing FR libraries for these positions. Each FR module position has defined 4 nucleotide non-palindromic fusion sites (indicated by numbers 1111 to 8888) allocated to the 5' and the 3' end of the respective DNA fragment. The respective fusion sites are generated by the Type IIS restriction enzyme flanking them (Bsal). They are combined with the CDR sequences in assembly reaction that results in a combinatorial library. Shown are three distinct examples of the resulting HC-library
**B)** In a second assembly the sub-libraries for heavy chain (HC-Library) and light chain (LC-Library) are assembled into a final combinatorial IgG library for functional characterization.

### DESCRIPTION OF THE INVENTION

The provided humanization method of this invention solves the problem for increased need for a high-throughput humanization platform technology compatible with established antibody engineering workflows. More precisely, higher throughput humanization is enabled by empirical recombination of human germline encoded frameworks with non-human CDRs via seamless Golden Gate cloning in a vector expression system and screening for variants displaying binding activities and for biophysical properties similar to those of the non-human parental antibody, of which the CDRs originate from.

The present invention relates to methods of re-engineering or re-shaping an antibody from a first species, wherein the re-engineered or re-shaped antibody does not elicit undesired immune response in a second species, and the re-engineered or re-shaped antibody retains substantially the same antigen binding-ability of the antibody from the first species. A combinatorial library comprising the CDRs of the antibody from the first species fused in frame with framework regions from a bank of framework regions derived from a second species can be constructed and screened for the desired modified antibody. Cells may comprise, contain or be engineered to express the nucleic acid sequences described herein. A method of producing a humanized heavy chain variable region as described herein may comprise expressing the nucleotide sequence encoding a humanized heavy chain variable region in a cell described herein. A method of producing a humanized light chain variable region may comprise expressing the nucleotide sequence encoding a humanized light chain variable region in a cell described herein. A method of producing a humanized antibody that immunospecifically binds to an antigen may comprise expressing the nucleic acid sequence(s) encoding the humanized antibody contained in the cell described herein.

The present invention provides a method of producing a humanized antibody that immunospecifically binds to an antigen, said method comprising: (a) synthesizing a plurality of polynucleotide sequences each comprising a nucleotide sequence encoding a humanized heavy chain variable region, said nucleotide sequence produced by fusing together a nucleic acid sequence encoding a human heavy chain framework region 1, a nucleic acid sequence encoding a heavy chain CDR1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a nonhuman donor antibody heavy chain variable region that immunospecifically binds said antigen and each heavy chain framework region nucleic acid sequence is from a subbank comprising a plurality of nucleic acid sequences encoding said human heavy chain framework region from human germline framework sequences and/or framework regions from functional human antibody sequences; (b) introducing the polynucleotide sequences into a population of cells each containing a polynucleotide sequence encoding a light chain variable region; and (c) expressing the polynucleotide sequences encoding the heavy chain variable region and the light chain variable region, characterized in that the nucleic acid sequences in step (a) are fused together by a step comprising the sequential or simultaneous activities of a type IIS restriction enzyme and a DNA ligase (the step is called "Type IIS cloning method" or "Golden Gate cloning method").

In another embodiment the present invention provides a method of producing a humanized antibody that immunospecifically binds to an antigen, said method comprising: (a) synthesizing a plurality of polynucleotide sequences each comprising a nucleotide sequence encoding a humanized light chain variable region, said nucleotide sequence produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a nonhuman donor antibody light chain variable region that immunospecifically binds said antigen and each light chain framework region nucleic acid sequence is from a subbank comprising a plurality of nucleic acid sequences encoding said human light chain framework region from human germline framework sequences and/or framework regions from functional human antibody sequences; (b) introducing the polynucleotide sequences into a population of cells each containing a polynucleotide sequence encoding a heavy chain variable region and (c) expressing the polynucleotide sequences encoding the heavy chain variable region and the light chain variable region, characterized in that the nucleic acid sequences in step (a) are fused together by a step comprising the sequential or simultaneous activities of a type IIS restriction enzyme and a DNA ligase (the step is called "Type IIS cloning method" or "Golden Gate cloning method").

In another embodiment the present invention provides a method of producing a humanized antibody that immunospecifically binds to an antigen, said method comprising: (a) synthesizing a plurality of first polynucleotide sequences each comprising a nucleotide sequence encoding a humanized heavy chain variable region, said nucleotide sequence produced by fusing together a nucleic acid sequence encoding a human heavy chain framework region 1, a nucleic acid sequence encoding a heavy chain CDR1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a non-human donor antibody heavy chain variable region that immunospecifically binds said antigen and each heavy chain framework region nucleic acid sequence is from a sub-bank comprising a plurality of nucleic acid sequences encoding said heavy chain framework region from human germline framework sequences and/or framework regions from functional human antibody sequences; (b) synthesizing a plurality of second polynucleotide sequences each comprising a nucleotide sequence encoding a humanized light chain variable region, said nucleotide sequence produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a non-human donor antibody light chain variable region that immunospecifically binds said antigen and each light chain framework region nucleic acid sequence is from a sub-bank comprising a plurality of nucleic acid sequences encoding said human light chain framework region from human germline framework sequences and/or framework regions from functional human antibody sequences; (c) introducing the nucleic acid sequences generated in steps (a) and (b) into a population of cells; and (d) expressing the nucleotide sequences encoding the heavy chain variable region and the light chain variable region, characterized in that the nucleic acid sequences in step (a) and/or (b) are fused together by a step comprising the sequential or simultaneous activities of a type IIS restriction enzyme and a DNA ligase (the step is called "Type IIS cloning method" or "Golden Gate cloning method").

In all embodiments described supra the recognition sites of the type IIS restriction enzyme are preferably placed to the far 5' and 3' end of the DNA fragments that are supposed to be assembled in opposite orientation as it is known from "Golden Gate cloning". Engler et al. PLoS One. 2009;4(5):e5553.

In another embodiment the present invention provides a method of producing a humanized antibody that immunospecifically binds to an antigen which further comprises, after the step of expressing the nucleotide sequences as described above, a step of screening for an antibody that immunospecifically binds to the antigen.

In a preferred embodiment of this invention the method of producing a humanized antibody that immunospecifically binds to an antigen the type IIS restriction enzyme is selected from the group consisting of Bsal, Bpil, Esp31, Sap, Lgul. In a more preferred embodiment the type IIS restriction enzyme is Bsal.

In a preferred embodiment of this invention the method of producing a humanized antibody that immunospecifically binds to an antigen the DNA ligase (EC 6.5.1.1) is a T4 DNA ligase.

The described invention allows for uniformed screening of HC/LC paired FR-CDR variant libraries.

In a preferred embodiment the method described supra uses standardized generic FR libraries (e.g. libraries for FR-H1, FR-H2, FR-H3, FR-H4, FR-L1, FR-L2, FR-L3, and/or FR-L4) that can be recycled for multiple projects, and are combined with non-PCR methods to avoid uncontrolled recombination events. Furthermore the libraries do not have unwanted sequence deviations that originate from DNA oligo and PCR-mistakes because the FR sequence libraries are sequence validated (EP 1869192 B1 Wu, et. al, describes the occurrence of unwanted side-mutations and the requirement for repairing final variants).

In contrast, the prior art uses oligo based shuffling procedures requiring de-novo generation of FR libraries for each intended humanization project.

By using sequence validated FR modules instead of non-validated DNA-oligo collections no sequence errors are introduced which base on error prone oligo synthesis. The quality of the final humanization DNA constructs is further increased by a non-PCR based assembly of said sequence validated FR. Here no additional PCR derived sequence deviations are introduced, no construct bias based on unequal primer annealing and no PCR-based recombination of homologue FRs occurs.

In a preferred embodiment in the methods of producing a humanized antibody that immunospecifically binds to an antigen described supra use defined and unique overhangs allocated to each sequence validated module type (FR1 to FR4 and CDR1 to CDR3). One non limiting example is provided within the EXAMPLES. This allows that CDRs originating from different organisms are compatible with the validated FR module sets. The defined and unique overhangs are compatible with the type IIS restriction enzyme used for the type IIS restriction enzyme and a DNA ligase cloning method (Golden gate method).

Because the cloning strategy is determined by the standardized nucleotide overhangs allocated to each functional module class, it has not to be adjusted between different humanization projects, (Fig. 1) and examples. This also allows that the validated FR modules can be recycled between different humanization projects.

The present invention provides methods of humanization an antibody (i.e. a donor antibody) by fusing together nucleic acid sequences encoding CDRs in frame with nucleic acid sequences encoding framework regions, wherein at least one CDR is from the donor antibody and at least one framework region is from a sub-bank of framework regions (e.g. a sub-bank sequences encoding some or all known human germline light chain FR1 :frameworks) using Type IIS based cloning methods.

The methods of the present invention may be utilized for the production of a humanized antibody from a non-human species, wherein the humanized antibody may not elicit undesired immune response retains substantially the same or better antigen binding characteristics of the antibody from the first species. Accordingly, the present invention provides a method resulting in humanized antibodies comprising one or more CDRs from a first species and at least one FR from humans. In some embodiments the present invention provides a method resulting in a humanized antibody comprises at least one, or at least two, or at least three, or at least four, or at least five, or six CDRs from a first species. In some embodiments, a humanized antibody of the invention comprises at least one, or at least two, or at least three, or at least four, or at least five, or at least six, or at least seven, or eight frameworks from humans.

Human framework genes might be selected from the publicly available pool of antibody germline genes and/or framework regions from functional human antibody sequences.

A plurality of different FR, framework region modules (FR1 to FR4) each comprising defined and unique overhangs is called a framework region sub-bank. These framework region sub-banks are used in the methods described above.

For example for the light chain the 46 human germline kappa light chain genes: A1, A10, A11, A14, A17, A18, A19, A2, A20, A23, A26, A27, A3, A30, A5, A7, B2, B3, L1, L10, L11, L12, L14, L15, L16, L18, L19, L2, L20, L22, L23, L24, L25, L4/18a, L5, L6, L8, L9, O1, O11, 012, 014, 018, 02, 04, and 08 (Schable et al. Biol. Chem. Hoppe Seyler 374 (1993) 1001-1022; Brensing-Kuppers et al. Gene 191 (1997) 173-18129) for the 1st, 2nd, and 3^{rd} frameworks, and five human germline Jκ sequences: Jκ1, Jκ2, Jκ3, Jκ4, and Jκ5 (Hieter et al. J. Biol. Chem. 257 (1982) 1516-1522) the 4th framework.

For example for the lambda light chain genes about 30 functional VL lambda genes comprising eleven subfamilies: IGLV1-36, IGLV1-40, IGLV1-41, IGLV1-44, IGLV1-47, IGLV1-50, IGLV1-51, IGLV1-62, IGLV2-5, IGLV2-8, IGLV2-11, IGLV2-14, IGLV2-18, IGLV2-23, IGLV2-33, IGLV2-34, IGLV2-NL1, IGLV3-1, IGLV3-9, IGLV3-10, IGLV3-12, IGLV3-13, IGLV3-16, IGLV3-19, IGLV3-21, IGLV3-22, IGLV3-25, IGLV3-27, IGLV3-31, IGLV3-32, IGLV4-3, IGLV4-60, IGLV4-69, IGLV5-37, IGLV5-39, IGLV5-45, IGLV5-48, IGLV5-52, IGLV6-57, IGLV7-43, IGLV7-46, IGLV8-61, IGLV9-49, IGLV10-54, IGLV11-55 are known (Pallares et al.; 1998 Exp. Clin. Immunogenet. 15(1):8-18) for the 1^{st}, 2^{nd} and 3^{rd} framework, and seven human germline Jλ sequences IGLJ1, IGLJ2, IGLJ3, IGLJ4, IGLJ5, IGLJ6, IGLJ7, (Lefranc, Exp Clin Immunogenet 2001;18:242-254) for the 4^{th} framework.

For example for the heavy chain the 44 human germline heavy chain genes: VH1-18, VH1-2, VH1-24, VH1-3, VH1-45, VH1-46, VH1-58, VH1-69, VH1-8, VH2-26, VH2-5, VH2-70, VH3-11, VH3-13, VH3-15, VH3-16, VH3-20, VH3-21, VH3- 23, VH3-30, VH3-33, VH3-35, VH3-38, VH3-43, VH3-48, VH3-49, VH3-53, VH3-64, VH3-66, VH3-7, VH3-72, VH3-73, VH3-74, VH3-9, VH4-28, VH4-31, VH4-34, VH4-39, VH4-4, VH4-59, VH4-61, VH5-51, VH6-1, and VH7-81 (Matsuda et al. J. Exp. Med. 188 (1998) 1973-1975) for the 1st, 2nd, and 3rd frameworks, and the six human germline JH sequences: JH1, JH2, JH3, JH4, JH5, and JH6 (Ravetch et al. Cell 27 (3 Pt 2) (1981) 583-591) for the 4th framework.

As shown in Fig.1 in a first step combinatorial sub-libraries for VH and/or VL are constructed by fusing in frame non-human CDRs with corresponding human framework regions of the generic FR sub-libraries (e.g. libraries for FR-H1, FR-H2, FR-H3, FR-H4, FR-L1, FR-L2, FR-L3, and/or FR-L4) using defined and unique overhangs allocated to each sequence validated module type (FR1 to FR4 and CDR1 to CDR3). In a second step the sub-libraries for heavy chain (HC-Lib) and light chain (LC-Lib) are assembled into a final full IgG expression combinatorial library for functional characterization as described below. The defined and unique overhangs are compatible with the type IIS restriction enzyme used for the type IIS restriction enzyme and a DNA ligase cloning method (Golden gate method).

### Expression of the Combinatorial Libraries and selection of humanized antibodies

The combinatorial libraries can be expressed using any methods known in the art, including but not limited to bacterial expression system, mammalian expression system, transcription/translation coupled cell free systems, and in vitro ribosomal display system.

In preferred embodiments, the present invention encompasses the use of phage vectors to express the combinatorial libraries. Phage vectors have particular advantages of providing a means for screening a very large population of expressed display proteins and thereby locate one or more specific clones that code for a desired binding activity.

The use of phage display vectors to express a large population of antibody molecules are well known in the art and will not be reviewed in detail herein. The method generally involves the use of a filamentous phage (phagemid) surface expression vector system for cloning and expressing antibody species of a library. The isolation of a particular vector capable of expressing an antibody binding site of interest involves the introduction of the dicistronic expression vector able to express the phagemid display protein into a host cell permissive for expression of filamentous phage genes and the assembly of phage particles. Typically, the host is E. coli. Thereafter, a helper phage genome is introduced into the host cell containing the phagemid expression vector to provide the genetic complementation necessary to allow phage particles to be assembled. The resulting host cell is cultured to allow the introduced phage genes and display protein genes to be expressed, and for phage particles to be assembled and shed from the host cell. The shed phage particles are then harvested (collected) from the host cell culture media and screened for desirable antibody binding properties. Typically, the harvested particles are "panned" for binding with a preselected antigen. The strongly binding particles are then collected, and individual species of particles are clonally isolated and further screened for binding to the antigen. Phages which produce a binding site of desired antigen binding specificity are selected. After phage selection, the antibody coding regions from the phage can be isolated and used to generate whole antibodies or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')2 fragments can also be employed using methods known in the art.

The expressed combinatorial libraries can be screened for binding to the antigen recognized by the donor antibody using any methods known in the art. Preferably, a phage display library constructed and expressed is screened for binding to the antigen recognized by the donor antibody, and the phage expressing VH and/or VL domain with significant binding to the antigen can be isolated from a library using the conventional screening techniques. The phage particles shed from host cells are harvested (collected) from the host cell culture media and screened for desirable antibody binding properties. Typically, the harvested particles are "panned" for binding with a preselected antigen. The strongly binding particles are then collected, and individual species of particles are clonally isolated and further screened for binding to the antigen. Preferably, selected phage particles are recovered and used to infect fresh bacteria before recovering the desired nucleic acids.

BIAcore kinetic analysis can be used to determine the binding on and off rates (K_{d}) of antibodies of the invention to a specific antigen. BIAcore kinetic analysis comprises analyzing the binding and dissociation of an antigen from chips with immobilized antibodies on their surface. The binding affinity of an antibody (including a scFv or other molecule comprising, or alternatively consisting of, antibody fragments or variants thereof) to an antigen and the off-rate of an antibody-antigen interaction can be determined by competitive binding assays.

Preferably, ELISA is used as a secondary screening on supernatant prepared from bacterial culture expressing Fab fragments in order to confirm the clones identified.

Once one or more nucleic acids encoding a humanized antibody or fragment thereof with desired binding activity are selected, the nucleic acid can be recovered by standard techniques known in the art.

Once a nucleic acid encoding an antibody molecule or a heavy or light chain of an antibody, or fragment thereof (preferably, containing the heavy or light chain variable region) has been obtained, the vector for the production of the antibody molecule may be produced by recombinant DNA technology using techniques well known in the art. Thus, methods for preparing a protein by expressing a nucleic acid encoding an antibody are described herein. Methods which are well known to those skilled in the art can be used to construct expression vectors containing antibody coding sequences and appropriate transcriptional and translational control signals. These methods include, for example, in vitro recombinant DNA techniques, synthetic techniques, and in vivo genetic recombination. Replicable vectors may comprise a nucleotide sequence encoding an antibody molecule of the invention, a heavy or light chain of an antibody, a heavy or light chain variable domain of an antibody or a fragment thereof, or a heavy or light chain CDR, operably linked to a promoter. The expression of an antibody molecule, a heavy or light chain of an antibody, a heavy or light chain variable domain of an antibody or a fragment thereof, or a heavy or light chain CDR can be regulated by a constitutive promoter. Alternatively, the expression of an antibody molecule, a heavy or light chain of an antibody, a heavy or light chain variable domain of an antibody or a fragment thereof, or a heavy or light chain CDR is regulated by an inducible promoter.

The expression vector is transferred to a host cell by conventional techniques and the transfected cells are then cultured by conventional techniques to produce an antibody. Thus, host cells may contain a polynucleotide encoding an antibody or fragments thereof, or a heavy or light chain thereof, or portion thereof, or a single chain antibody, operably linked to a heterologous promoter. Preferably, for the expression of double-chained antibodies, vectors encoding both the heavy and light chains may be co-expressed in the host cell for expression of the entire immunoglobulin molecule, as detailed below.

### DNA molecules of the invention

The present invention also relates to the DNA molecules that encode an antibody of the invention or antigen-binding fragment thereof. These sequences are optimized in certain cases for mammalian expression. DNA molecules of the invention are not limited to the sequences disclosed herein, but also include variants thereof. DNA variants within the invention may be described by reference to their physical properties in hybridization. The skilled worker will recognize that DNA can be used to identify its complement and, since DNA is double stranded, its equivalent or homolog, using nucleic acid hybridization techniques. It also will be recognized that hybridization can occur with less than 100% complementarity. However, given appropriate choice of conditions, hybridization techniques can be used to differentiate among DNA sequences based on their structural relatedness to a particular probe. For guidance regarding such conditions see, Sambrook et al., 1989 *supra* and Ausubel et al., 1995 (Ausubel, F. M., Brent, R., Kingston, R. E., Moore, D. D., Sedman, J. G., Smith, J. A., & Struhl, K. eds. (1995). Current Protocols in Molecular Biology. New York: John Wiley and Sons).

Structural similarity between two polynucleotide sequences can be expressed as a function of "stringency" of the conditions under which the two sequences will hybridize with one another. As used herein, the term "stringency" refers to the extent that the conditions disfavor hybridization. Stringent conditions strongly disfavor hybridization, and only the most structurally related molecules will hybridize to one another under such conditions. Conversely, non-stringent conditions favor hybridization of molecules displaying a lesser degree of structural relatedness. Hybridization stringency, therefore, directly correlates with the structural relationships of two nucleic acid sequences.

Hybridization stringency is a function of many factors, including overall DNA concentration, ionic strength, temperature, probe size and the presence of agents which disrupt hydrogen bonding. Factors promoting hybridization include high DNA concentrations, high ionic strengths, low temperatures, longer probe size and the absence of agents that disrupt hydrogen bonding. Hybridization typically is performed in two phases: the "binding" phase and the "washing" phase.

### Functionally Equivalent DNA Variants

Yet another class of DNA variants within the scope of the invention may be described with reference to the product they encode. These functionally equivalent polynucleotides are characterized by the fact that they encode the same peptide sequences due to the degeneracy of the genetic code.

It is recognized that variants of DNA molecules provided herein can be constructed in several different ways. For example, they may be constructed as completely synthetic DNAs. Methods of efficiently synthesizing oligonucleotides are widely available. See Ausubel *et al*., section 2.11, Supplement 21 (1993). Overlapping oligonucleotides may be synthesized and assembled in a fashion first reported by Khorana et al., J. Mol. Biol. 72:209-217 (1971); *see also* Ausubel *et al.*, *supra*, Section 8.2. Synthetic DNAs preferably are designed with convenient restriction sites engineered at the 5' and 3' ends of the gene to facilitate cloning into an appropriate vector.

As indicated, a method of generating variants is to start with one of the DNAs disclosed herein and then to conduct site-directed mutagenesis. *See* Ausubel et al., *supra*, chapter 8, Supplement 37 (1997). In a typical method, a target DNA is cloned into a single-stranded DNA bacteriophage vehicle. Single-stranded DNA is isolated and hybridized with an oligonucleotide containing the desired nucleotide alteration(s). The complementary strand is synthesized and the double stranded phage is introduced into a host. Some of the resulting progeny will contain the desired mutant, which can be confirmed using DNA sequencing. In addition, various methods are available that increase the probability that the progeny phage will be the desired mutant. These methods are well known to those in the field and kits are commercially available for generating such mutants.

### Recombinant DNA constructs and expression

The present invention further provides recombinant DNA constructs comprising one or more of the nucleotide sequences encoding the preferred antibodies of the present invention. The recombinant constructs of the present invention can be used in connection with a vector, such as a plasmid, phagemid, phage or viral vector, into which a DNA molecule encoding an antibody of the invention or antigen-binding fragment thereof or variant thereof is inserted.

An antibody, antigen binding portion, or variant thereof provided herein can be prepared by recombinant expression of nucleic acid sequences encoding light and heavy chains or portions thereof in a host cell. To express an antibody, antigen binding portion, or variant thereof recombinantly a host cell can be transfected with one or more recombinant expression vectors carrying DNA fragments encoding the light and/or heavy chains or portions thereof such that the light and heavy chains are expressed in the host cell. Standard recombinant DNA methodologies are used to prepare and/or obtain nucleic acids encoding the heavy and light chains, incorporate these nucleic acids into recombinant expression vectors and introduce the vectors into host cells, such as those described in Sambrook, Fritsch and Maniatis (eds.), Molecular Cloning; A Laboratory Manual, Second Edition, Cold Spring Harbor, N.Y., (1989), Ausubel, F. M. et al. (eds.) Current Protocols in Molecular Biology, Greene Publishing Associates, (1989) and in U.S. Pat. No. 4,816,397 by Boss et al..

In addition, the nucleic acid sequences encoding variable regions of the heavy and/or light chains can be converted, for example, to nucleic acid sequences encoding full-length antibody chains, Fab fragments, or to scFv. The VL- or VH-encoding DNA fragment can be operatively linked, (such that the amino acid sequences encoded by the two DNA fragments are in-frame) to another DNA fragment encoding, for example, an antibody constant region or a flexible linker. The sequences of human heavy chain and light chain constant regions are known in the art (see e.g., Kabat, E. A., el al. (1991) Sequences of Proteins of Immunological Interest, Fifth Edition, U.S. Department of Health and Human Services, NIH Publication No. 91-3242) and DNA fragments encompassing these regions can be obtained by standard PCR amplification.

To create a polynucleotide sequence that encodes a scFv, the VH- and VL-encoding nucleic acids can be operatively linked to another fragment encoding a flexible linker such that the VH and VL sequences can be expressed as a contiguous single-chain protein, with the VL and VH regions joined by the flexible linker (see e.g., Bird et al. (1988) Science 242:423-426; Huston et al. (1988) Proc. Natl. Acad. Sci. USA 85:5879-5883; McCafferty et al., Nature (1990) 348:552-554).

To express the antibodies, antigen binding fragments thereof or variants thereof standard recombinant DNA expression methods can be used (see, for example, Goeddel; Gene Expression Technology. Methods in Enzymology 185, Academic Press, San Diego, Calif. (1990)). For example, DNA encoding the desired polypeptide can be inserted into an expression vector which is then transfected into a suitable host cell. Suitable host cells are prokaryotic and eukaryotic cells. Examples for prokaryotic host cells are e.g. bacteria, examples for eukaryotic hosts cells are yeasts, insects and insect cells, plants and plant cells, transgenic animals, or mammalian cells. In some embodiments, the DNAs encoding the heavy and light chains are inserted into separate vectors. In other embodiments, the DNA encoding the heavy and light chains is inserted into the same vector. It is understood that the design of the expression vector, including the selection of regulatory sequences is affected by factors such as the choice of the host cell, the level of expression of protein desired and whether expression is constitutive or inducible.

Therefore, an embodiment of the present invention are also host cells comprising the vector or a nucleic acid molecule, whereby the host cell can be a higher eukaryotic host cell, such as a mammalian cell, a lower eukaryotic host cell, such as a yeast cell, and may be a prokaryotic cell, such as a bacterial cell.

Another embodiment of the present invention is a method of using the host cell to produce an antibody and antigen binding fragments, comprising culturing the host cell under suitable conditions and recovering said antibody.

Therefore another embodiment of the present invention is the production of the antibodies according to this invention with the host cells of the present invention and purification of these antibodies to at least 95% homogeneity by weight.

### Bacterial Expression

Useful expression vectors for bacterial use are constructed by inserting a DNA sequence encoding a desired protein together with suitable translation initiation and termination signals in operable reading phase with a functional promoter. The vector will comprise one or more phenotypic selectable markers and an origin of replication to ensure maintenance of the vector and, if desirable, to provide amplification within the host. Suitable prokaryotic hosts for transformation include but are not limited to *E. coli, Bacillus subtilis, Salmonella typhimurium* and various species within the genera Pseudomonas, Streptomyces, and Staphylococcus.

Bacterial vectors may be, for example, bacteriophage-, plasmid- or phagemid-based. These vectors can contain a selectable marker and a bacterial origin of replication derived from commercially available plasmids typically containing elements of the well-known cloning vector pBR322 (ATCC 37017). Following transformation of a suitable host strain and growth of the host strain to an appropriate cell density, the selected promoter is de-repressed/induced by appropriate means (e.g., temperature shift or chemical induction) and cells are cultured for an additional period. Cells are typically harvested by centrifugation, disrupted by physical or chemical means, and the resulting crude extract retained for further purification.

In bacterial systems, a number of expression vectors may be advantageously selected depending upon the use intended for the protein being expressed. For example, when a large quantity of such a protein is to be produced, for the generation of antibodies or to screen peptide libraries, for example, vectors which direct the expression of high levels of fusion protein products that are readily purified may be desirable.

Therefore, an embodiment of the present invention is an expression vector comprising a nucleic acid sequence encoding for the novel antibodies of the present invention.

Antibodies of the present invention or antigen-binding fragments thereof or variants thereof include naturally purified products, products of chemical synthetic procedures, and products produced by recombinant techniques from a prokaryotic host, including, for example, *E. coli, Bacillus subtilis, Salmonella typhimurium* and various species within the genera Pseudomonas, Streptomyces, and Staphylococcus, preferably, from *E. coli* cells.

### Mammalian Expression

Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. Expression of the antibodies may be constitutive or regulated (e.g. inducible by addition or removal of small molecule inductors such as Tetracyclin in conjunction with Tet system). For further description of viral regulatory elements, and sequences thereof, see e.g., U.S. 5,168,062 by Stinski, U.S. 4,510,245 by Bell et al. and U.S. 4,968,615 by Schaffner et al.. The recombinant expression vectors can also include origins of replication and selectable markers (see e.g., U.S. 4,399,216, 4,634,665 and U.S. 5,179,017). Suitable selectable markers include genes that confer resistance to drugs such as G418, puromycin, hygromycin, blasticidin, zeocin/bleomycin or methotrexate or selectable marker that exploit auxotrophies such as Glutamine Synthetase (Bebbington et al., Biotechnology (N Y). 1992 Feb;10(2):169-75), on a host cell into which the vector has been introduced. For example, the dihydrofolate reductase (DHFR) gene confers resistance to methotrexate, neo gene confers resistance to G418, the bsd gene from Aspergillus terreus confers resistance to blasticidin, puromycin N-acetyl-transferase confers resistance to puromycin, the Sh ble gene product confers resitance to zeocin, and resistance to hygromycin is conferred by the E. coli hygromycin resistance gene (hyg or hph). Selectable markers like DHFR or Glutamine Synthetase are also useful for amplification techniques in conjunction with MTX and MSX.

Transfection of the expression vector into a host cell can be carried out using standard techniques such as electroporation, nucleofection, calcium-phosphate precipitation, lipofection, polycation-based transfection such as polyethlylenimine (PEI)-based transfection and DEAE-dextran transfection.

Suitable mammalian host cells for expressing the antibodies, antigen binding fragments thereof or variants thereof provided herein include Chinese Hamster Ovary (CHO cells) such as CHO-K1, CHO-S, CHO-K1SV [including dhfr- CHO cells, described in Urlaub and Chasin, (1980) Proc. Natl. Acad. Sci. USA 77:4216-4220 and Urlaub et al., Cell. 1983 Jun;33(2):405-12, used with a DHFR selectable marker, e.g., as described in R. J. Kaufman and P. A. Sharp (1982) Mol. Biol. 159:601-621; and other knockout cells exemplified in Fan et al., Biotechnol Bioeng. 2012 Apr;109(4):1007-15], NS0 myeloma cells, COS cells, HEK293 cells, HKB11 cells, BHK21 cells, CAP cells, EB66 cells, and SP2 cells.

Expression might also be transient or semi-stable in expression systems such as HEK293, HEK293T, HEK293-EBNA, HEK293E, HEK293-6E, HEK293-Freestyle, HKB11, Expi293F, 293EBNALT75, CHO Freestyle, CHO-S, CHO-K1, CHO-K1SV, CHOEBNALT85, CHOS-XE, CHO-3E7 or CAP-T cells (for instance Durocher et al., Nucleic Acids Res. 2002 Jan 15;30(2):E9).

In some embodiments, the expression vector is designed such that the expressed protein is secreted into the culture medium in which the host cells are grown. The antibodies, antigen binding fragments thereof or variants thereof can be recovered from the culture medium using standard protein purification methods.

### Purification

Antibodies of the invention or antigen-binding fragments thereof or variants thereof can be recovered and purified from recombinant cell cultures by well-known methods including, but not limited to ammonium sulfate or ethanol precipitation, acid extraction, Protein A chromatography, Protein G chromatography, anion or cation exchange chromatography, phospho-cellulose chromatography, hydrophobic interaction chromatography, affinity chromatography, hydroxylapatite chromatography and lectin chromatography. High performance liquid chromatography ("HPLC") can also be employed for purification. See, e.g., Colligan, Current Protocols in Immunology, or Current Protocols in Protein Science, John Wiley & Sons, NY, N.Y., (1997-2001), e.g., Chapters 1, 4, 6, 8, 9, 10, each entirely incorporated herein by reference.

Antibodies of the present invention or antigen-binding fragments thereof or variants thereof include naturally purified products, products of chemical synthetic procedures, and products produced by recombinant techniques from an eukaryotic host, including, for example, yeast, higher plant, insect and mammalian cells. Depending upon the host employed in a recombinant production procedure, the antibody of the present invention can be glycosylated or can be non-glycosylated. Such methods are described in many standard laboratory manuals, such as Sambrook, supra, Sections 17.37-17.42; Ausubel, supra, Chapters 10, 12, 13, 16, 18 and 20.

In preferred embodiments, the antibody is purified (1) to greater than 95% by weight of antibody as determined e.g. by the Lowry method, UV-Vis spectroscopy or by by SDS-Capillary Gel electrophoresis (for example on a Caliper LabChip GXII, GX 90 or Biorad Bioanalyzer device), and in further preferred embodiments more than 99% by weight, (2) to a degree sufficient to obtain at least 15 residues of N-terminal or internal amino acid sequence, or (3) to homogeneity by SDS-PAGE under reducing or non-reducing conditions using Coomassie blue or, preferably, silver stain. Isolated naturally occurring antibody includes the antibody in situ within recombinant cells since at least one component of the antibody's natural environment will not be present. Ordinarily, however, isolated antibody will be prepared by at least one purification step.

### EXAMPLES

The present invention is further described by the following examples. The examples are provided solely to illustrate the invention by reference to specific embodiments. These exemplifications, while illustrating certain specific aspects of the invention, do not portray the limitations or circumscribe the scope of the disclosed invention.

All examples were carried out using standard techniques, which are well known and routine to those of skill in the art, except where otherwise described in detail. Routine molecular biology techniques of the following examples can be carried out as described in standard laboratory manuals, such as Sambrook et al., Molecular Cloning: A Laboratory Manual, 2nd Ed.; Cold Spring Harbor Laboratory Press, Cold Spring Harbor, N.Y., 1989.

### Example : Generation of a IgG1 FR shuffled library

### Molecular biology reagents

Restriction enzymes used were purchased from New England Biolabs (Ipswitch, MA) and Fermentas (Burlington, Canada). T4 DNA ligase was purchased from Promega (Fitchburg, WI). Plasmid DNA preparations were made by using the QIAprep Spin miniprep Kit (Qiagen, Hilden, Germany) following the manufacturer protocol. Plasmid DNA concentration was measured using a Nano Drop® Spectrophotometer ND-1000 (Peqlab, Erlangen, Germany).

### Construction of the FR recyclable module compilations

DNA fragments encoding defined human FR sequences (1st, 2nd, 3rd and 4th FR from heavy and light chain germline sequences) are selected from the publicly available pool of human antibody germline genes. For example for the light chain the 46 human germline kappa light chain genes: A1, A10, A11, A14, A17, A18, A19, A2, A20, A23, A26, A27, A3, A30, A5, A7, B2, B3, L1, L10, L11, L12, L14, L15, L16, L18, L19, L2, L20, L22, L23, L24, L25, L4/18a, L5, L6, L8, L9, O1, O11, O12, O14, O18, O2, O4, and O8 (Schable et al. Biol. Chem. Hoppe Seyler 374 (1993) 1001-1022; Brensing-Kuppers et al. Gene 191 (1997) 173-18129) for the 1st, 2nd, and 3rd frameworks, and five human germline Jκ sequences: Jκ1, Jκ2, Jκ3, Jκ4, and Jκ5 (Hieter et al. J. Biol. Chem. 257 (1982) 1516-1522) the 4th framework.

For example for the lambda light chain genes about 30 functional VL lambda genes comprising eleven subfamilies: IGLV1-36, IGLV1-40, IGLV1-41, IGLV1-44, IGLV1-47, IGLV1-50, IGLV1-51, IGLV1-62, IGLV2-5, IGLV2-8, IGLV2-11, IGLV2-14, IGLV2-18, IGLV2-23, IGLV2-33, IGLV2-34, IGLV2-NL1, IGLV3-1, IGLV3-9, IGLV3-10, IGLV3-12, IGLV3-13, IGLV3-16, IGLV3-19, IGLV3-21, IGLV3-22, IGLV3-25, IGLV3-27, IGLV3-31, IGLV3-32, IGLV4-3, IGLV4-60, IGLV4-69, IGLV5-37, IGLV5-39, IGLV5-45, IGLV5-48, IGLV5-52, IGLV6-57, IGLV7-43, IGLV7-46, IGLV8-61, IGLV9-49, IGLV10-54, IGLV11-55 are known (Pallares et al.; 1998 Exp. Clin. Immunogenet. 15(1):8-18) for the 1^{st}, 2nd and 3rd framework, and seven human germline JI sequences IGLJ1, IGLJ2, IGLJ3, IGLJ4, IGLJ5, IGLJ6, IGLJ7, (Lefranc, Exp Clin Immunogenet 2001;18:242-254) for the 4th framework.

For example for the heavy chain the 44 human germline heavy chain genes: VH1-18, VH1-2, VH1-24, VH1-3, VH1-45, VH1-46, VH1-58, VH1-69, VH1-8, VH2-26, VH2-5, VH2-70, VH3-11, VH3-13, VH3-15, VH3-16, VH3-20, VH3-21, VH3- 23, VH3-30, VH3-33, VH3-35, VH3-38, VH3-43, VH3-48, VH3-49, VH3-53, VH3-64, VH3-66, VH3-7, VH3-72, VH3-73, VH3-74, VH3-9, VH4-28, VH4-31, VH4-34, VH4-39, VH4-4, VH4-59, VH4-61, VH5-51, VH6-1, and VH7-81 (Matsuda et al. J. Exp. Med. 188 (1998) 1973-1975) for the 1^{st}, 2nd, and 3rd frameworks, and the six human germline JH sequences: JH1, JH2, JH3, JH4, JH5, and JH6 (Ravetch et al. Cell 27 (3 Pt 2) (1981) 583-591) for the 4th framework.

All FR sequences can be ordered as defined sequences subcloned into standard cloning vectors like pUC in order to allow long time storage and easy maintenance of the FR modules. Each FR sequence belonging to a defined class (FR-H1, FR-H2, FR-H3, FR-H4, FR-L1, FR-L2, FR-L3, FR-L4) is flanked 5' and 3' by a class specific nucleotide overhang and a Bsal Type IIS restriction enzyme recognition site generating the FR class specific overhang. The specific nucleotide overhangs are designed to encode for highly conserved amino acid residues separating FR and CDR regions in human and non-human species. An example FR/CDR set for HC and LC is listed in table 1 (HC) and table 2 (LC).

**Table 1: Example for FR module design and corresponding CDRs for an antibody heavy chain. Fusion sites underlined. Type IIS restriction site labelled in bold.**

| **Name** | **SEQ ID NO:** | **Sequence** |
|---|---|---|
| FR-H1-3-23 | 1 | ggtctctccatgaagcacctgtggttctttctgctgctggtggccgctccc agatgggtgctgtctgaagtgcagctggtggaatctggcggaggactggtt caacctggcggctctctgagactgtcttgtgccgccagcggcagagacc |
| CDR-H1 | 2 | **ggtctct**cggcttcaacatcaaggacacctacatccactgg**agagacc** |
| FR-H2-3-23 | 3 | **ggtctct**ctgggtccgacaggcccctggcaaaggacttgaatgg**agagacc** |
| CDR-H2 | 4 | ggtctctatggtcgccagaatctaccccaccaacggctacaccagttaca gagacc |
| FR-H3-3-23 | 5 | ggtctctttacgccgactctgtgaagggcagattcaccatcagcgccgaca ccagcaagaacaccgcctacctgcagatgaacagcctgagagccgaggaca ccgccgtgtactactgcagagacc |
| CDR-H3 | 6 | ggtctctctgc tctagatggggaggcgacggcttctacgccatggattattggggccagggc accagagacc |
| FR-JH1-IgG1-Fc | 7 | ggtctctcacccctcgtgaccgtgtcatcagccagcaccaagggccccagcg tgttccctctggcccctagcagcaagagcacatctggcggaacagccgccc tgggctgcctcgtgaaggactactttcccgagcccgtgaccgtgtcctgga actctggcgctctgacaagcggcgtgcacacctttccagccgtgctgcaga gcagcggcctgtactctctgagcagcgtcgtgacagtgcccagcagctctc tgggcacccagacctacatctgcaacgtgaaccacaagcccagcaacacca aggtggacaagaaggtggaacccaagagctgcgacaagacccacacctgtc cccccttgtcctgcccccgaactgctgggaggcccttccgtgttcctgttcc ccccaaagcccaaggacaccctgatgatcagccggacccccgaagtgacct gcgtggtggtggatgtgtcccacgaggaccctgaagtgaagttcaattggt acgtggacggcgtggaagtgcacaacgccaagaccaagcctagagaggaac agtacaacagcacctaccgggtggtgtccgtgctgacagtgctgcaccagg actggctgaacggcaaagagtacaagtgcaaggtgtccaacaaggccctgc ctgcccccatcgagaaaaccatcagcaaggccaagggccagccccgcgaac cccaggtgtacacactgcccccaagcagggacgagctgaccaagaaccagg tgtccctgacctgtctcgtgaaaggcttctaccccctccgatatcgccgtgg aatgggagagcaacggccagcccgagaacaactacaagaccaccccccctg tgctggacagcgacggctcattcttcctgtacagcaagctgaccgtggaca agtcccggtggcagcagggcaacgtgttcagctgcagcgtgatgcacgagg ccctgcacaaccactacacccagaagtccctgagcctgagccctggctaat agagacc |

**Table 2: Example for FR module design and corresponding CDRs for an antibody light chain. Fusion sites underlined Type IIS restriction site labelled in bold.**

| **Name** | **SEQ ID NO:** | **Sequence** |
|---|---|---|
| FR-L1-K1-39 | 8 | ggtctctccatggtgctgcagacccaggtgttcatcagcctgctgctg tggatcagcggcgcctatggcgacatccagatgacacagagccctagc agcctgtctgccagcgtgggagacagagtgaccatcacatgcagagac c |
| CDR-L1 | 9 | ggtctctatgc     agagccagccaggacgtgaacacagccgtggct tggtagagacc |
| FR-L2-K1-39 | 10 | ggtctcttggt atcagcagaagcctggcaaggcccctaagctgctgat ctatagagacc |
| CDR-L2 | 11 | **ggtctct**ctat agcgccagctttctgtacagcggt**agagacc** |
| FR-L3-K1-39 | 12 | ggtctctcggt gtgcccagcagattcagcggctctagaagcggcaccgacttcaccctg accataagcagtctgcagcccgaggacttcgccacctactactgtaga gacc |
| CDR-L3 | 13 | ggtctctctgtcagcagcactacaccacacctccaacc tttgagagacc |
| FR-JLK2-IgG1-Fc | 14 | ggtctcttttg gccagggcaccaaggtggaaatcaaacgaaccgtggccgctcccagcg tgttcatcttcccacctagcgacgagcagctgaagtccggcacagcct ctgtcgtgtgcctgctgaacaacttctacccccgcgaggccaaggtgc agtggaaggtggacaatgccctgcagagcggcaacagccaggaaagcg tgaccgagcaggacagcaaggactccacctacagcctgagcagcaccc tgaccctgagcaaggccgactacgagaagcacaaggtgtacgcctgcg aagtgacccaccagggcctgtctagccccgtgaccaagagcttcaacc ggggcgagtgttaatagagacc |

### Generation of CDR sequences of interest compatible to the FR recyclable module compilations

CDR sequences of interest can be generated by standard procedures known in the art like PCR or defined from identified antibody sequences and ordered as subcloned nucleotide sequence. The CDR sequences have to be flanked 5'and 3' by overhangs compatible to the adjacent FR modules to be usable. E.g CDR-H1 has 5' the 3' overhang of FR-H1 and 3' the 5' overhang of the FR-H2.

### Generation of light chain FR libraries

The respective CDR fragments CDR-L1, CDR-L2 and CDR-L3 can be combined in a Golden Gate cloning reaction according to Engler et al. 2009 with a selected set of FR-L1, FR-L2, FR-L3, FR-L4, a plasmid encoding the constant part, a destination plasmid pL1-LC and the respective CDR-L1, CDR-L2 and CDR-L3 encoding plasmids. CDR sequences of interest can be generated by standard procedures known in the art like PCR or defined from identified antibody sequences and ordered as subcloned nucleotide sequence. The CDR sequences have to be flanked 5'and 3' by overhangs compatible to the adjacent FR modules. E.g CDR-H1 has 5' the 3' overhang of FR-L1 and 3' the 5' overhang of the FR-L2. All DNA modules have to be combined in a Golden Gate cloning reaction which has to be setup with approximately 30 fmol (∼100 ng for a 5 kb plasmid) of each plasmid., Promega ligation buffer, 10 U of the respective restriction enzyme (Bsal, Bpil), 10 U high concentrated T4 DNA ligase, in a 20 µl volume. The reaction will be incubated for 4 hours at 37 °C, 5 min 50 °C and 5 min 80 °C. The mix will be added to 50 µl electro competent Top10 cells, incubated for 15-30 min on ice and transformed by electroporation. Clones will be analyzed by restriction analysis, colony PCR and optionally by sequencing and represent the LC-Library.

### Generation of HC chain FR libraries

The respective CDR fragments CDR-H1, CDR-H2 and CDR-H3 can be combined in a Golden Gate cloning reaction according to Engler et al. 2009 with a selected set of FR-H1, FR-H2, FR-H3, FR-H4 encoding plasmids, an IgG constant part encoding plasmid, a destination plasmid pL1-HC, the respective CDR-H1, CDR-H2 and CDR-H3 sequences of interest. CDR sequences of interest can be generated by standard procedures known in the art like PCR or defined from identified antibody sequences and ordered as subcloned nucleotide sequence. The CDR sequences have to be flanked 5'and 3' by overhangs compatible to the adjacent FR modules. E.g. CDR-H1 has 5' the 3' overhang of FR-H1 and 3' the 5' overhang of the FR-H2. All DNA modules have to be combined in a Golden Gate cloning reaction which has to be setup with approximately 30 fmol (∼100 ng for a 5 kb plasmid) of each plasmid., Promega ligation buffer, 10 U of the respective restriction enzyme (Bsal, Bpil), 10 U high concentrated T4 DNA ligase, in a 20 µl volume. The reaction will be incubated for 4 hours at 37 °C, 5 min 50 °C and 5 min 80 °C. The mix will be added to 50 µl electro competent Top10 cells, incubated for 15-30 min on ice and transformed by electroporation. Clones will be analyzed by restriction analysis, colony PCR and optionally by sequencing and represent the HC-Library.

### Generation of HC/LC antibody FR libraries

The antibody heavy chain FR libraries (HC-Library) and antibody light chain FR libraries (LC-Library) are combined into a final expression library in a single plasmid backbone in order to maintain coupling of LC and HC in the later functional antibody characterization steps. Therefore both libraries are combined in a further Golden Gate reaction into the final expression plasmid. In this-three-fragment cloning reaction the library plasmids HC-Library, LC-Library and the final destination plasmid pL2-1 are combined in equimolar ratios of approximately 30 fmol (∼100 ng for a 5 kb plasmid) of each plasmid., Promega ligation buffer, 10 U of the respective restriction enzyme (Bsal, Bpil), 10 U high concentrated T4 DNA ligase, in a 20 µl volume. The reaction will be incubated for 4 hours at 37 °C, 5 min 50 °C and 5 min 80 °C. The mix will be added to 50 µl electro competent Top10 cells, incubated for 15-30 min on ice and transformed by electroporation. Clones will be analyzed by restriction analysis, colony PCR and optionally by sequencing and represent the humanized antibody Library.

## Claims

1. A method of producing a humanized antibody that immunospecifically binds to an antigen, said method comprising: (a) synthesizing a plurality of polynucleotide sequences each comprising a nucleotide sequence encoding a humanized heavy chain variable region, said nucleotide sequence produced by fusing together a nucleic acid sequence encoding a human heavy chain framework region 1, a nucleic acid sequence encoding a heavy chain CDR1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a nonhuman donor antibody heavy chain variable region that immunospecifically binds said antigen and each heavy chain framework region nucleic acid sequence is from a subbank comprising a plurality of nucleic acid sequences encoding said human heavy chain framework region from human germline framework sequences and/or framework regions from functional human antibody sequences; (b) introducing the polynucleotide sequences into a population of cells each containing a polynucleotide sequence encoding a light chain variable region; and (c) expressing the polynucleotide sequences encoding the heavy chain variable region and the light chain variable region, **characterized in that** the nucleic acid sequences in step (a) are fused together by a step comprising the sequential or simultaneous activities of a type IIS restriction enzyme and a DNA ligase.

2. A method of producing a humanized antibody that immunospecifically binds to an antigen, said method comprising: (a) synthesizing a plurality of polynucleotide sequences each comprising a nucleotide sequence encoding a humanized light chain variable region, said nucleotide sequence produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a nonhuman donor antibody light chain variable region that immunospecifically binds said antigen and each light chain framework region nucleic acid sequence is from a subbank comprising a plurality of nucleic acid sequences encoding said human light chain framework region from human germline framework sequences and/or framework regions from functional human antibody sequences; (b) introducing the polynucleotide sequences into a population of cells each containing a polynucleotide sequence encoding a heavy chain variable region and (c) expressing the polynucleotide sequences encoding the heavy chain variable region and the light chain variable region, **characterized in that** the nucleic acid sequences in step (a) are fused together by a step comprising the sequential or simultaneous activities of a type IIS restriction enzyme and a DNA ligase.

3. A method of producing a humanized antibody that immunospecifically binds to an antigen, said method comprising: (a) synthesizing a plurality of first polynucleotide sequences each comprising a nucleotide sequence encoding a humanized heavy chain variable region, said nucleotide sequence produced by fusing together a nucleic acid sequence encoding a human heavy chain framework region 1, a nucleic acid sequence encoding a heavy chain CDR1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a non-human donor antibody heavy chain variable region that immunospecifically binds said antigen and each heavy chain framework region nucleic acid sequence is from a subbank comprising a plurality of nucleic acid sequences encoding said heavy chain framework region from human germline framework sequences and/or framework regions from functional human antibody sequences; (b) synthesizing a plurality of second polynucleotide sequences each comprising a nucleotide sequence encoding a humanized light chain variable region, said nucleotide sequence produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a non-human donor antibody light chain variable region that immunospecifically binds said antigen and each light chain framework region nucleic acid sequence is from a sub-bank comprising a plurality of nucleic acid sequences encoding said human light chain framework region from human germline framework sequences and/or framework regions from functional human antibody sequences; (c) introducing the nucleic acid sequences generated in steps (a) and (b) into a population of cells; and (d) expressing the nucleotide sequences encoding the heavy chain variable region and the light chain variable region, **characterized in that** the nucleic acid sequences in step (a) and/or (b) are fused together by a step comprising the sequential or simultaneous activities of a type IIS restriction enzyme and a DNA ligase.

4. A method of producing a humanized antibody that immunospecifically binds to an antigen according to any one of claims 1 to 3, wherein the DNA ligase is a T4 DNA ligase.

5. A method of producing a humanized antibody that immunospecifically binds to an antigen according to any one of claims 1 to 4 wherein the type IIS restriction enzyme is selected from the group consisting of Bsal, Bpil, Esp31, Sap, Lgul.

6. A method of producing a humanized antibody that immunospecifically binds to an antigen according to any one of claims 1 to 5 wherein the type IIS restriction enzyme is Bsal.

7. A method of producing a humanized antibody that immunospecifically binds to an antigen according to any of claims 1 to 6 which further comprises, after the step of expressing the nucleotide sequences, a step of screening for an antibody that immunospecifically binds to the antigen.

8. A method of producing a humanized antibody that immunospecifically binds to an antigen according to any of claims 1 to 6 which further comprises, after the step of expressing the nucleotide sequences, a step of purification of the humanized antibody.


SEQUENCE LISTING

<110>  Bayer Pharma AG

<120>  One step Antibody Humanization by Golden Gate Based Germline
       Framework Region Shuffling

<130>  BHC 15 1 004

<160>  14

<170>  PatentIn version 3.5

<210>  1
<211>  151
<212>  DNA
<213>  Artificial Sequence

<220>
<223>  FR-H1-3-23

<400>  1
ggtctctcca tgaagcacct gtggttcttt ctgctgctgg tggccgctcc cagatgggtg      60

ctgtctgaag tgcagctggt ggaatctggc ggaggactgg ttcaacctgg cggctctctg     120

agactgtctt gtgccgccag cggcagagac c                                    151

<210>  2
<211>  48
<212>  DNA
<213>  Artificial Sequence

<220>
<223>  CDR-H1

<400>  2
ggtctctcgg cttcaacatc aaggacacct acatccactg gagagacc                   48

<210>  3
<211>  51
<212>  DNA
<213>  Artificial Sequence

<220>
<223>  FR-H2-3-23

<400>  3
ggtctctctg ggtccgacag gcccctggca aaggacttga atggagagac c               51

<210>  4
<211>  57
<212>  DNA
<213>  Artificial Sequence

<220>
<223>  CDR-H2

<400>  4
ggtctctatg ggtcgccaga atctacccca ccaacggcta caccagttac agagacc         57

<210>  5
<211>  126
<212>  DNA
<213>  Artificial Sequence

<220>
<223>  FR-H3-3-23

<400>  5
ggtctcttta cgccgactct gtgaagggca gattcaccat cagcgccgac accagcaaga      60

acaccgccta cctgcagatg aacagcctga gagccgagga caccgccgtg tactactgca     120

gagacc                                                                126


<210>  6
<211>  72
<212>  DNA
<213>  Artificial Sequence

<220>
<223>  CDR-H3

<400>  6
ggtctctctg ctctagatgg ggaggcgacg gcttctacgc catggattat tggggccagg      60

gcaccagaga cc                                                          72


<210>  7
<211>  1027
<212>  DNA
<213>  Artificial Sequence

<220>
<223>  FR-JH1-IgG1-Fc

<400>  7
ggtctctcac cctcgtgacc gtgtcatcag ccagcaccaa gggccccagc gtgttccctc      60

tggcccctag cagcaagagc acatctggcg gaacagccgc cctgggctgc ctcgtgaagg     120

actactttcc cgagcccgtg accgtgtcct ggaactctgg cgctctgaca agcggcgtgc     180

acacctttcc agccgtgctg cagagcagcg gcctgtactc tctgagcagc gtcgtgacag     240

tgcccagcag ctctctgggc acccagacct acatctgcaa cgtgaaccac aagcccagca     300

acaccaaggt ggacaagaag gtggaaccca agagctgcga caagacccac acctgtcccc     360

cttgtcctgc ccccgaactg ctgggaggcc cttccgtgtt cctgttcccc ccaaagccca     420

aggacaccct gatgatcagc cggacccccg aagtgacctg cgtggtggtg gatgtgtccc     480

acgaggaccc tgaagtgaag ttcaattggt acgtggacgg cgtggaagtg cacaacgcca     540

agaccaagcc tagagaggaa cagtacaaca gcacctaccg ggtggtgtcc gtgctgacag     600

tgctgcacca ggactggctg aacggcaaag agtacaagtg caaggtgtcc aacaaggccc     660

tgcctgcccc catcgagaaa accatcagca aggccaaggg ccagccccgc gaaccccagg 720

tgtacacact gcccccaagc agggacgagc tgaccaagaa ccaggtgtcc ctgacctgtc 780

tcgtgaaagg cttctacccc tccgatatcg ccgtggaatg ggagagcaac ggccagcccg 840

agaacaacta caagaccacc ccccctgtgc tggacagcga cggctcattc ttcctgtaca 900

gcaagctgac cgtggacaag tcccggtggc agcagggcaa cgtgttcagc tgcagcgtga 960

tgcacgaggc cctgcacaac cactacaccc agaagtccct gagcctgagc cctggctaat 1020

agagacc 1027

<210> 8
<211> 145
<212> DNA
<213> Artificial Sequence

<220>
<223> FR-L1-K1-39

<400> 8
ggtctctcca tggtgctgca gacccaggtg ttcatcagcc tgctgctgtg gatcagcggc 60

gcctatggcg acatccagat gacacagagc cctagcagcc tgtctgccag cgtgggagac 120

agagtgacca tcacatgcag agacc 145

<210> 9
<211> 55
<212> DNA
<213> Artificial Sequence

<220>
<223> CDR-L1

<400> 9
ggtctctatg cagagccagc caggacgtga acacagccgt ggcttggtag agacc 55

<210> 10
<211> 59
<212> DNA
<213> Artificial Sequence

<220>
<223> FR-L2-K1-39

<400> 10
ggtctcttgg tatcagcaga agcctggcaa ggcccctaag ctgctgatct atagagacc 59

<210> 11
<211> 42
<212> DNA
<213> Artificial Sequence

<220>
<223> CDR-L2

<400>  11
ggtctctcta tagcgccagc tttctgtaca gcggtagaga cc                          42


<210>  12
<211>  111
<212>  DNA
<213>  Artificial Sequence

<220>
<223>  FR-L3-K1-39

<400>  12
ggtctctcgg tgtgcccagc agattcagcg gctctagaag cggcaccgac ttcaccctga       60

ccataagcag tctgcagccc gaggacttcg ccacctacta ctgtagagac c               111


<210>  13
<211>  49
<212>  DNA
<213>  Artificial Sequence

<220>
<223>  CDR-L3

<400>  13
ggtctctctg tcagcagcac tacaccacac ctccaacctt tgagagacc                   49


<210>  14
<211>  369
<212>  DNA
<213>  Artificial Sequence

<220>
<223>  FR-JLK2-IgG1-Fc

<400>  14
ggtctctttt ggccagggca ccaaggtgga aatcaaacga accgtggccg ctcccagcgt       60

gttcatcttc ccacctagcg acgagcagct gaagtccggc acagcctctg tcgtgtgcct      120

gctgaacaac ttctaccccc gcgaggccaa ggtgcagtgg aaggtggaca atgccctgca      180

gagcggcaac agccaggaaa gcgtgaccga gcaggacagc aaggactcca cctacagcct      240

gagcagcacc ctgaccctga gcaaggccga ctacgagaag cacaaggtgt acgcctgcga      300

agtgacccac cagggcctgt ctagccccgt gaccaagagc ttcaaccggg gcgagtgtta      360

atagagacc                                                              369